(19) Europäisches Patentamt
European Patent Office
Office européen des brevets

(11) Publication number: **0 011 858**
**B1**

(12) **EUROPEAN PATENT SPECIFICATION**

(45) Date of publication of patent specification: **18.05.83**

(21) Application number: **79104742.6**

(22) Date of filing: **28.11.79**

(51) Int. Cl.³: **C 07 B 23/00,**
C 07 D 211/14,
C 07 D 207/06,
C 07 C 87/28,
C 07 C 87/60,
C 07 C 103/20,
A 61 K 43/00, A 61 K 49/02
//C07C25/02, C07C121/78

(54) **Radioiodine containing amines, their preparation and their use as brain imaging agents.**

(30) Priority: **29.11.78 US 964561**

(43) Date of publication of application:
**11.06.80 Bulletin 80/12**

(45) Publication of the grant of the patent:
**18.05.83 Bulletin 83/20**

(84) Designated Contracting States:
**CH DE FR GB IT**

(56) References cited:
**CA - A - 1 040 217**
**FR - A - 2 154 486**
**US - A - 4 048 297**

**ANALYTICAL CHEMISTRY, vol. 27, no. 12, December 1955, pages 1895, 1898 Washington, U.S.A.**
**W. M. STOKES et al.: "Chromatographic separation and quantitative Estimation of Jodine-131-Labeled Derivatives of Sterols, Amines, Acids and Aldehydes"**

(73) Proprietor: **F. HOFFMANN-LA ROCHE & CO. Aktiengesellschaft**
**CH-4002 Basel (CH)**

(72) Inventor: **Baldwin, Ronald Martin**
**3828 Anza Street**
**San Francisco, California (US)**
Inventor: **Lin, Tz-Hong**
**3337 Kemper Road**
**Fremont, California (US)**
Inventor: **Winchell, Harry Saul**
**No. 1 Via Oneg**
**Lafayette, California (US)**

(74) Representative: **Lederer, Franz, Dr. et al,**
**Patentanwälte Dr. Franz Lederer Reiner F. Meyer**
**Lucile-Grahn-Strasse 22**
**D-8000 München 80 (DE)**

(56) References cited:
**JOURNAL OF NUCLEAR MEDICINE, vol. 20, no. 2, February 1979, pages 155—158**
**New York, U.S.A.**
**D. M. WIELAND et al.: "Imaging the Adrenal Medulla with an 1—131-labeled Antiadrenergic Agent"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 011 858 B1

Courier Press, Leamington Spa, England

# Radioiodine containing amines, their preparation and their use as brain imaging agents

In the Canadian Patent Specification No. 1.040.217 quaternary ammonium salts and methods for their use are described, inter alia the compound I-125-o-iodobenzyltrimethyl-ammonium chloride. In the US Patent Specification 4.048.297 novel quaternary ammonium salts, compositions and methods for their use are described, inter alia the compound I-125-p-iodobenzyltrimethyl-amminium chloride.

The present invention, however, is concerned with radioiodinated amine compounds represented by the formula

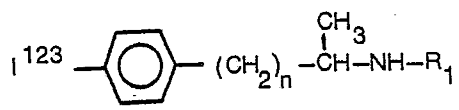

A

wherein n is 0 or 1 and $R_1$ is hydrogen or isopropyl and their pharmaceutically acceptable acid addition salt.

The compounds of formula A are useful for imaging of the brain. As will therefore be appreciated, the compounds of formula A demonstrate rapid accumulation in the brain indicative of an ability to penetrate the so-termed "blood/brain barrier."

The compounds of formula A demonstrate a marked superiority in stability of the iodine-carbon bond in vivo. The stability of the carbon-iodine bond in vivo in combination with the ability to penetrate the blood/brain barrier enable the compounds of formula A to demonstrate rapid localization of the radioiodine in the brain following intravenous administration. In addition to the primary localization in the brain, the compounds of formula A also show localization in the heart, adrenals and pancreas.

The stability of the iodine label in vivo; i.e., in the brain, and the ability to penetrate the blood/brain barrier are distinct advantages of the compounds of formula A in their use as brain imaging agents.

The pharmaceutically acceptable acid additional salts of the compounds of formula A include salts with both inorganic and organic pharmaceutically acceptable acids. Suitable inorganic acids include, for example, hydrochloric acid, hydrobromic acid, hydroiodic acid, sulfuric acid and the like. Suitable organic acids include, for example, benzoic acid, acetic acid, citric acid, lactic acid and the like.

The radioiodinated amines in accordance with the present invention include:

I-123-p-iodo-α-methylbenzylamine;
I-123-N-isopropyl-p-iodo-α-methylbenzylamine;
I-123-R-p-iodoamphetamine;
I-123-S-p-iodoamphetamine;
I-123-R-N-isopropyl-p-iodoamphetamine; and
I-123-S-N-isopropyl-p-iodoamphetamine.

The compounds of formula A can be prepared by methods recognized in the art. For example, a compound of formula A may be prepared by reacting a compound represented by the formula

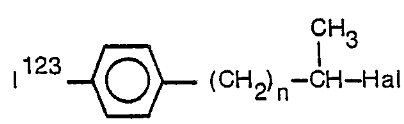

B

wherein n is as defined above and Hal is chloro, bromo oder iodo, with an amino represented by the formula

$$H_2N—R_1 \qquad C$$

wherein $R_1$ is a previously defined.

For the above reaction, the amine may be utilized to create alkaline conditions, i.e. added in excess, or the reactants may be present in stoichiometric quantities and the reaction carried out in the presence of a suitable base such as, for example, sodium hydroxide, potassium hydroxide and the like. The reaction is carried out at ambient temperature or with heating, e.g. at a temperature up to about 200°C, preferably at about 25°C.

Compounds of formula A, wherein $R_1$ is isopropyl may also be prepared by reacting a compound of formula A wherein $R_1$ is hydrogen with acetone to form the corresponding Schiff base and then reducing the Schiff base chemically or catalytically.

The reduction of the Schiff base as described above may be carried out either chemically or catalytically by methods well known in the art. A preferred chemical method for reduction of the Schiff base utilizes an alkali metal cyanoborohydride as the reducing agent in a suitable organic solvent such

as, for example, methanol, dioxane and tetrahydrofuran, with methanol being preferred.

Finally, compounds of formula A may be prepared by the iodination of an amine having the amine group protected, e.g. by an alkanoyl group, i.e. a compound represented by the formula

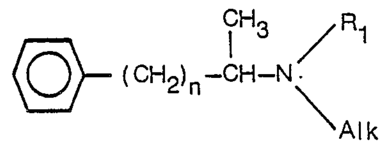

D

wherein $R_1$ and n are as defined above and Alk is an alkanoyl group, and subsequently removing the protecting alkanoyl group by conventional methods. The iodination is carried out by conventional methods such as utilizing iodine in the presence of an oxidizing agent such as iodic acid or nitric acid at elevated temperatures. The alkanoyl protecting group can be removed, for example, by hydrolyzing in acid medium under reflux.

The above reactions can be carried out utilizing the respective compounds wherein I is stable iodine and not iodine 123 under the same conditions. The resulting compounds wherein I is stable iodine; i.e. the cold compounds, are then exchange labeled with I-123.

For the exchange radiolabeling process, an inorganic salt of iodine 123, preferably an alkali metal salt and most preferably the sodium salt is utilized. The salt is heated with the cold compound of formula A; i.e. a compound corresponding to formula A but wherein I is stable iodine and not iodine 123 for from about 1/4 to about 2 hours. The exchange radiolabeling is carried out in the presence of an inert organic solvent such as, for example, a lower alcohol or those named above.

The radiolabeled compound of formula A is then dissolved in a suitable solvent; e.g. ethyl ether, and washed with aqueous sodium bisulfite solution to remove unexchanged radioiodine. The product is then extracted into hydrochloric acid and washed with a suitable water immiscible organic solvent; e.g. ethyl ether, then basified with a suitable aqueous base and re-extracted into the organic solvent. The organic solution is then evaporated to dryness to obtain a pure labeled product.

The radiolabeling procedure described above may likewise be carried out starting with an acid addition salt of a compound of formula A by treating it with a suitable base and then extracting the free amine with a suitable organic solvent such as those named above.

Intermediate compounds such as, for example, those of formula B above can be radiolabeled in a like manner and then reacted as discussed above to form the corresponding compound of formula A. Many of the cold compounds of formulae A through D are known compounds.

In addition to the partition procedure described above, the radiolabeled compounds of formula A and the intermediates can be purified by preparative thin layer chromatography and liquid chromatography utilizing procedures well known in the art.

The compounds of formula A can be rapidly prepared, e.g. by exchange radiolabeling. This is advantageous because of the criticality of time in the handling of radioisotopes which have a comparatively short half life such as iodine I-123.

The compounds of formula A as well as the corresponding cold compounds can be converted to their respective pharmaceutically acceptable acid addition salts by methods conventional in the art, for example, by direct reaction with the appropriate acid. The hydrochloride can be prepared, for example, by contacting a solution of the amine of formula A in a suitable organic solvent with hydrogen chloride gas and recovering the precipitated amine hydrochloride.

As stated above, the radioiodine containing compounds of the invention rapidly localize in the brain following intravenous administration. In most instances, a sufficient amount of the administered dose will accumulate in the brain within from about one to ten minutes to permit the taking of scintiphotos. The compounds of the invention will show meaningful presence in the brain for at least 15 minutes so that significant studies may be carried out. In addition to the brain, the compounds of formula A will also accumulate in myocardium, adrenals and pancreas to varying degrees.

The radioiodinated compounds of the subject invention may be administered in an aqueous or aqueous/alcoholic medium. Such media may also contain conventional pharmaceutical adjunct materials such as, for example, pharmaceutically acceptable salts to adjust the osmotic pressure, buffers, preservatives and the like.

A preferred vehicle for the parenteral administration of the compounds of formula A is normal saline which would contain from about 0.5% by weight to about 2% by weight of a suitable preservative.

The following examples further illustrate the invention. Unless otherwise noted, all temperatures are in degrees centigrade.

Example 1

In accordance with the procedure given below R-N-isopropyl-p-iodoamphetamine, Rf = 0.44, and

S-N-isopropyl-p-iodoamphetamine, Rf = 0.46 were prepared by reacting R-p-iodoamphetamine and S-p-iodoamphetamine, respectively, with acetone.

To a solution of 0.71 g of o-iodophenethylamine hydrochloride and 0.145 g acetone in 5 ml of methanol was added an equimolar amount, i.e. 0.139 g of $NaBH_3CN$. The mixture was stirred at room temperature for three days. The mixture was then adjusted to pH 1 with 2N hydrochloric acid and the methanol removed by purging the mixture with nitrogen. The residue was extracted with 15 ml of chloroform. The extract was diluted with 100 ml of ether and the precipitated amine hydrochloride collected by filtration.

Recrystallization of the product from methanol/ether yielded 0.244 g of N-isopropyl-o-iodophenethylamine, as a white crystalline solid, mp 159—160°, $R_f$ value = 0.28.

## Example 2

A mixture of 7.5 g R-N-acetylamphetamine, 15 ml glacial acetic acid, 5 ml iodine, 2.5 g iodic acid, 2.5 ml concentrated sulfuric acid and 2.5 ml carbon tetrachloride was heated under reflux for 17 hours. The mixture was cooled and diluted with 50 ml deionized water and 15 ml chloroform. The mixture was washed three times with basic sodium bicarbonate and then with deionized water until the washing solution was neutral to litmus paper. The chloroform was evaporated and the residue hydrolyzed by refluxing in 100 ml of 6N hydrochloric acid for 14 hours. The reaction mixture was cooled and the resulting crystals collected by suction filtration. The crystals were washed with hot chloroform and rendered basic with 50 ml of 4.5N sodium hydroxide. The product was extracted with 100 ml of ether which, upon evaporation, yielded 4.2 g of R-p-iodo-amphetamine, $R_f$ = 0.3.

By the same procedure, S-p-iodo-amphetamine was prepared, $R_f$ = 0.3.

## Example 3

The compounds listed in Table I of Example 4 were labelled in accordance with the following procedure:

In a clean pyrex test tube was placed 10 mcl of N-t-butyl-o-iodobenzylamine and 4.08 mCi I-123-NaI in acetone. The solvent was evaporated with a stream of nitrogen, 40 ml of ethanol was added and the tube sealed with a torch.

The tube was heated in an autoclave at 121° for one hour. The tube was opened and the mixture taken up in 2 ml of ether. The ether solution was washed with a mixture of 1 ml 0.1M potassium iodide, 1 ml 1% sodium bisulfite and 0.1 ml 1N sodium hydroxide, followed by 1 ml water and was extracted with 1 ml 1N hydrochloric acid. The resulting solution was washed with 1 ml ether, rendered alkaline with 1.2 ml 1N sodium hydroxide and extracted with 1 ml ether. The ether solution was washed with two 1 ml portions of water. The ether solution was found to contain 85% of the original activity. A control tube containing no substrate but otherwise treated in the same manner showed less than 1% of the original activity in the ether solution.

The ether solution was shaken with 1 ml saturated sodium chloride solution and dried over anhydrous sodium sulfate. The ether was evaporated with a nitrogen stream and the residue dissolved in 1 ml of 0.1N hydrochloric acid which was treated with 0.6 ml of 0.1N sodium hydroxide to obtain a final pH of 4.5. Radiochemical purity by radiochromatography was 100%.

The starting material N-t-butyl-o-iodobenzylamine is prepared as follows:

o-Iodobenzyl bromide was prepared by the procedure described by H. A. Sloviter, J. Am. Chem. Soc. *71*, p. 3360 (1949) for p-iodobenzyl bromide by starting with o-iodotoluene. The crude product was distilled at reduced pressure to yield a liquid product, bp. 85—92°/0.5 mm Hg, which crystallized spontaneously to yield a solid product having a mp of 42—49°.

o-Iodobenzyl chloride was prepared from o-iodobenzyl alcohol using thionyl chloride according to the procedure described by Brooks et al., Org. Syn.Coll. Vol. 3, p. 698 (1955). The crude product was distilled at reduced pressure to yield a clear, colorless liquid having a bp of 143°/4 mm Hg.

A total of 1.74 g of o-iodobenzyl bromide was stirred with 10 ml of t-butylamine for 18 hrs. at 25°C. The resulting fine white precipitate was collected by suction filtration and washed with 10 ml ether to yield 646 mg of t-butylamine hydrobromide. The filtrate was concentrated on a rotary evaporator to a viscous liquid which was dissolved in 25 ml ether and washed with three 20 ml portions of water and two 10 ml portions of 1N hydrochloric acid.

The combined acidic washings were basified with 5 ml 5N sodium hydroxide and extracted with 25 ml of ether. The ether solution was washed with four 10 ml portions of water, until the pH of the washing was less than 9, followed by 20 ml saturated sodium chloride solution. The ether solution was then dried with anhydrous magnesium sulfate, filtered and the solvent removed on a rotary evaporator to yield 1.36 g of colorless liquid which was purified by molecular distillation at 80°/0.25 mm to yield 1.11 g of N-t-butyl-o-iodobenzylamine, $R_f$ value of 0.31 on thin layer chromatography on silica gel 60 (1% $CH_3OH/CHCl_3$).

## Example 4

Bioassays were performed utilizing compounds from the foregoing examples labeled with I-123 in accordance with the method described in example 3. Female Sprague-Dawley rats weighing

approximately 150 g were anesthetized with sodium pentobarbital and were injected in a tail vein with from 0.05 to 1.0 mCi (in a volume of 0.2 to 0.5 ml) of the I-123-labeled compound. Two specimens were utilized for each test.

Two animals each were sacrificed at 5 minutes and 60 minutes post-injection, the tails discarded and the amount of activity in various organs determined. Each organ was counted at a standardized geometry with a sodium iodide crystal scintillation counter adjusted for the 159KeV emission of I-123. The organs were also weighed to one thousandth of a gram and the activity calculated as a percent of administered dose per gram of organ weight.

The ratio of brain-to-blood activity was calculated as a percent of administered dose per gram. The results for blood, brain and heart as well as the brain/blood ratio are reported in table I. For comparative purposes, I-123 antipyrine was utilized as a standard.

TABLE I

| Compound | Percent Dose / Gram | | | | Brain / Blood | | | |
|---|---|---|---|---|---|---|---|---|
| | Blood | | Brain | | Heart | | Ratio | |
| | T min. | 60 min. | 5 min. | 60 min. | 5 min. | 60 min. | 5 min. | 60 min. |
| I–123–R–p–iodoamphetamine | 0.15 | 0.12 | 1.38 | 2.07 | 2.43 | 1.09 | 10.60 | 18.50 |
| I–123–S–p–iodoamphetamine | 0.17 | 0.17 | 1.22 | 1.88 | 1.52 | 0.86 | 7.04 | 11.30 |
| I–123–R–N–isopropyl–p–iodo-amphetamine | 0.14 | 0.11 | 1.57 | 2.14 | 1.78 | 0.87 | 12.60 | 20.70 |
| I–123–S–N–isopropyl–p–iodo-amphetamine | 0.11 | 0.13 | 1.32 | 1.93 | 1.28 | 0.85 | 12.80 | 15.40 |
| I–123–iodoantipyrine [a] | 1.26 | 0.94 | 0.61 | 0.17 | 0.98 | 0.53 | 0.48 | 0.18 |

[a] Prepared according to J. Nucl. Med., *17*, 1093 (1976).

**Claims**

1. A radioiodinated amine compound represented by the formula

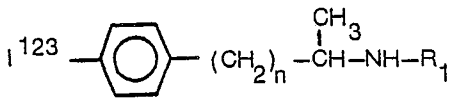 A

wherein n is 0 or 1 and $R_1$ is hydrogen or isopropyl, and pharmaceutically acceptable acid addition salts thereof.

2. I-123-R-N-isopropyl-p-iodoamphetamine.
3. I-123-S-N-isopropyl-p-iodoamphetamine.
4. A compound as claimed in any one of claims 1—3 as brain imaging agent.
5. A process for the preparation of a radioiodinated amine compound represented by the formula

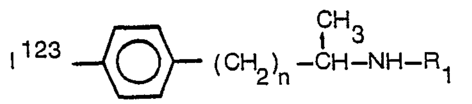 A

wherein n is 0 or 1 and $R_1$ is hydrogen or isopropyl, and pharmaceutically acceptable acid addition salts thereof which comprises labelling a compound corresponding to formula A but having a stable nonradioactive isotope of iodine, with iodine 123.

6. A process according to claim 5, wherein I-123-R-N-isopropyl-p-iodoamphetamine is prepared.
7. A process according to claim 5, wherein I-123-S-N-isopropyl-p-iodoamphetamine is prepared.

**Patentansprüche**

1. Eine radiojodierte Aminoverbindung, dargestellt durch die Formel

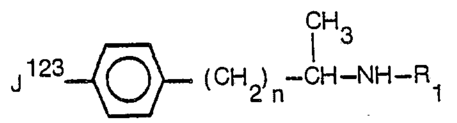 A

worin n 0 oder 1 und $R_1$ Wasserstoff oder Isopropyl ist, und pharmazeutische annehmbare Säureadditionssalze davon.

2. J-123-R-N-Isopropyl-p-jodamphetamin.
3. J-123-S-N-Isopropyl-p-jodamphetamin.
4. Eine Verbindung, wie sie in einem der Ansprüche 1—3 beansprucht wird, als Hirndarstellendes Mittel.
5. Verfahren zur Herstellung einer radiojodierten Aminoverbindung, dargestellt durch die Formel

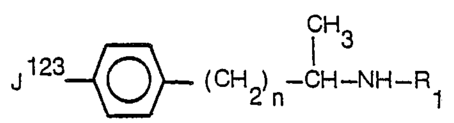 A

worin n 0 oder 1 und $R_1$ Wasserstoff oder Isopropyl ist, und pharmazeutisch annehmbare Säureadditionssalze davon, welches die Markierung einer Verbindung entsprechend der Formel A, die jedoch ein stabiles, nicht radioaktives Isotop von Jod hat, mid Jod 123 umfasst.

6. Verfahren gemäss Anspruch 5, bei dem Jod-123-R-N-isopropyl-p-jodamphetamin hergestellt wird.
7. Verfahren nach Anspruch 5, bei dem Jod-123-S-N-isopropyl-p-jodamphetamin hergestellt wird.

**Revendications**

1. Composé d'amine radio-iodée représenté par la formule:

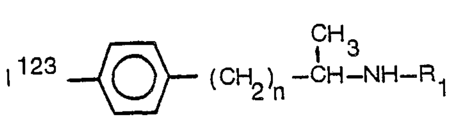 A

où n vaut 0 ou 1 et $R_1$ est un hydrogène ou un isopropyle, et leurs sels d'addition d'acides pharmaceutiquement acceptables.

2. I-123-R-N-isopropyl-p-iodoamphétamine.

3. I-123-S-N-isopropyl-p-iodoamphétamine.

4. Composé tel que revendiqué dans l'une quelconque des revendications 1 à 3 comme agent de visualisation du cerveau.

5. Procédé de préparation d'un composé d'amine radio-iodée représenté par la formule:

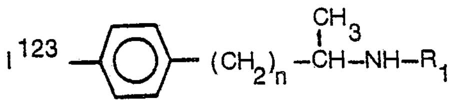

A

où n vaut 0 ou 1 et $R_1$ est un hydrogène ou un isopropyle, et de leurs sels d'addition d'acides pharmaceutiquement acceptables, dans lequel on marque un composé correspondant à la formule A, mais ayant un isotope non radioactif stable de l'iode, avec de l'iode 123.

6. Procédé selon la revendication 5 où l'on prépare la I-123-R-N-isopropyl-p-iodoamphétamine.

7. Procédé selon la revendication 5 où l'on prépare la I-123-S-N-isopropyl-p-iodoamphétamine.